# EUROPEAN PATENT SPECIFICATION

(11) **EP 4 181 941 B1**
(45) Date of publication and mention of the grant of the patent: **09.09.2026**
(21) Application number: 21842678.1
(22) Date of filing: 13.07.2021
(51) Int. Cl.: A61K 36/752, A61K 36/75, A61K 36/28, A61K 36/53, A61K 36/185, A61P 35/00, A23L 33/105, A61P 3/10, A61P 13/12, A61P 25/08, A61P 25/28

(54) **COMPOSITIONS INCLUDING A NEROLI HYDROSOL, A CHAMOMILE HYDROSOL AND A ROSEMARY HYDROSOL AND USES OF THE COMPOSITION**
ZUSAMMENSETZUNGEN, ENTHALTEND EIN NEROLI-HYDROSOL, EIN KAMILLEN-HYDROSOL UND EIN ROSMARIN-HYDROSOL, SOWIE VERWENDUNGEN DER ZUSAMMENSETZUNGEN
COMPOSITIONS COMPRENANT UN HYDROSOL DE NÉROLI, UN HYDROSOL DE CAMOMILLE ET UN HYDROSOL DE ROMARIN ET UTILISATIONS DE LA COMPOSITION

(30) Priority: 14.07.2020 HK 32020011192
(43) Date of publication of application: 24.05.2023
(73) Proprietor: Natur-Tech Pharmacal Co., Ltd, Kowloon, Hong Kong (HK)
(72) Inventor: LAI, Lily, Hong Kong (CN)
(74) Representative: Marks & Clerk LLP
(86) International application number: PCT/CN2021/106092
(87) International publication number: WO 2022/012551

(56) References cited:
- WO-A1-2008/017484
- WO-A1-2017/173993
- WO-A1-2017/218853
- CN-A- 101 773 488
- CN-A- 104 814 491
- CN-A- 106 578 199
- CN-A- 106 727 032
- KR-A- 20170 120 781
- KR-B1- 100 429 671
- KR-B1- 102 118 555
- US-A1- 2015 374 771
- MUHAMMAD AYAZ ET AL: "Neuroprotective and Anti-Aging Potentials of Essential Oils from Aromatic and Medicinal Plants", FRONTIERS IN AGING NEUROSCIENCE, vol. 9, 1 January 2017 (2017-01-01), CH, pages 1 - 16, XP055633940, ISSN: 1663-4365, DOI: 10.3389/fnagi.2017.00168
- CLARA DOBETSBERGER ET AL: "Actions of essential oils on the central nervous system: An updated review", FLAVOUR AND FRAGRANCE JOURNAL, WILEY, NEW YORK, NY, GB, vol. 26, no. 5, 18 February 2011 (2011-02-18), pages 300 - 316, XP071708542, ISSN: 0882-5734, DOI: 10.1002/FFJ.2045

## Description

### TECHNICAL FIELD

The present invention relates to a composition, in particular but not exclusively an oral composition, including a neroli hydrosol, a chamomile hydrosol and a rosemary hydrosol, for promoting the health condition of a subject. The invention also relates to a method of treating and/or reducing a subject's risk of suffering from a disorder.

### BACKGROUND

People having a hectic job or busy schedules generally suffer from considerable stress in life. The prolonged stress and bad habits may contribute to psychological heath issues and even more seriously a mental illness including depression, anxiety disorders, eating disorders, social withdrawal, etc. Depression is one of the common mental illnesses which can be serious as it can significantly affect how people feel and act in a negative way. Anxiety can also occur at the same time when the people feel stressed. Besides, mental illness can also lead to physical illness for example a person who is suffering from depression may be at risk of gastrointestinal disorder and cardiovascular disease. The immune system of people who have mental illness is generally vulnerable and is susceptible to other health issues including, but not limited to, diabetes, arthritis, autoimmune disorders, and even cancer.

A number of drugs have been developed to treat different types of psychological or mental illnesses. For example, antidepressants such as fluoxetine, citalopram, paroxetine, sertraline, fluvoxamine are commonly used selective serotonin reuptake inhibitors for alleviating symptoms associated with depression. However, these antidepressants may cause severe side effects such as nausea, headaches, diarrhea, and decreased sexual desire. Other existing drugs for treating depression include venlafaxine and duloxetine which may lead to increased blood pressure, headache and constipation.

Epilepsy is a common neurological disorder nowadays. According to the epidemiological statistics, the incidence of epilepsy each year is about 50-70 in every 100,000 people, and the prevalence is about 5‰. In addition, there are about more than 6 million patients with epilepsy in China; and there are 650,000 to 700,000 new patients with epilepsy every year. This brain disorder is characterized by the persistence of the permanent changes in brain that may increase the possibility of seizures in the future, and by the corresponding neurobiological, cognitive, psychological, and social dysfunctions.

The current drugs for epilepsy cannot effectively prevent and cure epilepsy, but can only reduce or stop seizures, and often require lifelong medication. However, most anti-epileptic drugs exhibit adverse reactions, such as insensitivity in certain patients, teratogenicity and drug resistance, and obvious side effects, which have negative impacts on patients' behavior and cognitive function, etc. This results in intermittent treatment and repeated illness, making it difficult for the patients to accept.

Diabetic nephropathy is the deterioration of proper functioning in the kidneys that results from having diabetes. Statistically, around 40% of people with diabetes develop nephropathy. It has been found that it is possible to prevent or delay diabetic nephropathy through control of both blood glucose and blood pressure levels.

To help lower blood pressure, drugs including angiotensin-converting enzyme (ACE) inhibitors and angiotensin receptor blockers (ARBs) are commonly used, as they have been shown to protect kidney function and prevent further damage while lowering blood pressure. However, ACE inhibitors often produce side effects of a dry cough, hyperkalemia, and angioedema, while ARBs often causes dizziness, hyperkalemia, and headache.

Accordingly, there remains a strong need for developing a composition and a method for promoting health particularly psychological and mental health, as well as a method for treating, preventing or reducing the risk of suffering from a physical illness associated with oxidative stress, epilepsy, and diabetic nephropathy, without significant or with less side effects.

### SUMMARY OF THE INVENTION

According to a first aspect of the present invention, there is provided a composition including a neroli hydrosol, a chamomile hydrosol and a rosemary hydrosol.

According to a second aspect of the present invention, there is provided a composition of the first aspect for use in treatment of or reducing the risk of suffering from a disorder.

According to a third aspect of the present invention, there is provided a composition of the first aspect for use in treatment of or reducing the risk of suffering from a disorder, wherein the disorder is selected from a group consisting of a neurodegenerative disorder caused by oxidative stress, epilepsy and diabetic nephropathy.

Without intending to be limited by theory, it is believed that the composition of the present invention may be effective for treating, preventing and/or reducing the risk of suffering from a disorder in particularly a neurodegenerative disorder associated with oxidative stress, epilepsy, and diabetic nephropathy. The composition is also suitable for promoting psychological health of a subject; and/or increasing dopamine levels. Without intending to be limited by theory, it is believed that the composition is capable of protecting neuronal cells under oxidative stress for example by minimizing the undesired influence triggered by oxidative stress.

### BRIEF DESCRIPTION OF THE DRAWINGS

Figure 1 shows two microscopic images of normal zebrafish transfected with tyrosine hydroxylase (TH)-GFP, in which the left image shows the GFP expression in the zebrafish and the right image was taken under light microscope.
Figure 2 shows two microscopic images of zebrafish transfected with TH-GFP followed by incubation with the composition of an embodiment of the present invention, in which the left image shows the GFP expression in the zebrafish and the right image was taken under light microscope.
Figure 3 shows two microscopic images of zebrafish transfected with TH-GFP followed by incubation with the diluted composition, in which the left image shows the GFP expression in the zebrafish and the right image was taken under light microscope.
Figure 4 is a bar chart showing the effect of the composition of an embodiment of the present invention on neurites in SH-SY5Y cells after 24-hour treatment.
Figure 5 is a bar chart showing the effect of the composition of an embodiment of the present invention on neurites in SH-SY5Y cells after 48-hour treatment.
Figure 6 is a bar chart showing the average length of neurites of SH-SY5Y cells treated with various concentrations of the composition of an embodiment of the present invention before and after oxidative stress challenge, in which the cells were pretreated for 24 hours before the challenge.
Figure 7 is a bar chart showing the average length of neurites of SH-SY5Y cells treated with various concentrations of the composition of an embodiment of the present invention before and after oxidative stress challenge, in which the cells were pretreated for 48 hours before the challenge.
Figure 8 shows the changes in the length of neurites after treating the cells for 12 hours, in which the cells were subjected to oxidative stress challenge before addition of the composition of an embodiment of the present invention.
Figure 9 shows the changes in the length of neurites after treating the cells for 24 hours, i.e. for another 12 hours after obtaining the results for plotting Figure 8.

### DETAILED DESCRIPTION OF THE INVENTION

Unless otherwise specifically provided, all tests herein are conducted at standard conditions which include a room and testing temperature of 25 °C, sea level (1 atm.) pressure, pH 7, and all measurements are made in metric units. Furthermore, all percentages, ratios, etc. herein are by weight, unless specifically indicated otherwise. It is understood that unless otherwise specifically noted, the materials compounds, chemicals, etc. described herein are typically commodity items and/or industry-standard items available from a variety of suppliers worldwide.

The term "hydrosol" as used herein refers to a liquid obtained by distillation particularly steam distillation of a part of a plant or the entire plant followed by a separation. It can be obtained in the same process of preparing an essential oil from a plant material. A hydrosol contains more water-soluble compounds but less aromatic compounds compared to the corresponding essential oil derived from the same plant material. Therefore it may be a clear mixture containing tiny droplets or particles suspended therein, while the droplets or particles may hardly be observed via naked eyes. A hydrosol may also be called a hydrolat, a hydrolate, a distillate, or a floral water. It would be appreciated that the hydrosol may be obtained via distilling flowers, leaves, barks, stems, roots, berries, fruits, wood, or other parts of the plant, not necessarily only flowers.

A neroli hydrosol as used herein refers to a hydrosol derived from orange blossom, and the orange blossom may be from, but not limited to, flowers from *Citrus aurantium subsp. amara* or *Bigaradia.*

A chamomile hydrosol as used herein refers to a hydrosol derived from *Matricaria chamomilla,* and/or *Chamarmelum nobile.*

A rosemary hydrosol as used herein refers to a hydrosol derived from *Rosmarinus officinalis.*

The term "oral pharmaceutical composition" used herein refers to a composition that is suitable for therapeutically and/or prophylactically treating or preventing a disease by administering to a subject orally, i.e. through an oral administration. Accordingly, an oral pharmaceutical composition of the present invention may be in the form of a dispersion, an emulsion, a suspension, a syrup, a soft gelatin capsule, and the like. In a preferred embodiment, the oral pharmaceutical composition is in the form of a dispersion, an emulsion, or a suspension with or without a pharmaceutically acceptable excipient. The pharmaceutically acceptable excipient includes a liquid filler, a lubricant, a diluent, a solvent or an encapsulating material, for carrying and delivering active ingredients of the oral pharmaceutical composition to an organ or a part of the body of the subject to exert the therapeutic effect.

The expression "effective amount" generally denotes an amount sufficient to produce therapeutically desirable results, wherein the exact nature of the result varies depending on the specific disorder which is treated. When the disorder is accompanied by inflammation, the result is usually a suppression or decrease of the expression or functional activity of inflammatory factors. The results may also be an increase of the expression or functional activity of anti-inflammatory factors. When the disorder is associated with a mental disorder such as depression and anxiety, the result can be an increase in dopamine level, an increase in happiness, improved sleep quality, and/or better appetite. When the disorder is associated with a neurodegenerative disorder, the result can be an increase in neuronal cells growth or development including extension in length of neurites, and/or delayed progression of the neurological conditions. It will be appreciated by a physician or veterinarian having ordinary skill in the art to determine and prescribe the effective amount of the composition particularly an oral pharmaceutical composition of the present invention. The physician or veterinarian will also capable of adjusting the effective amount of the composition according to the severity of the condition to be alleviated.

The composition of the invention may promote dopamine production, relieving discomfort and/or alleviating symptoms associated with physical or mental illness. The composition herein is believed to be effective in increasing the level of dopamine and have neuroprotective effect against oxidative stress.

It is believed that the psychological health of an individual patient can be improved by enhancing the production of dopamine as dopamine is a key reward neurotransmitter that can regulate normal behavior, motivation and emotion, and affects movement, memory and focus. In general, the increase in dopamine will result in a better mood and therefore it is useful in regulating the emotion of a subject.

The composition of the present invention comprises hydrosols derived from plants.

The skilled person in the art is aware of suitable methods for obtaining a hydrosol from a plant material. The plant material is a non-transgenic plant material and more preferably a plant material that is free of pesticides and additives. One possible way to obtain a hydrosol is by conducting a steam distillation. For example, a steam is generated to pass through the plant material and cause the plant material to release essential oil and other substances in the form of vapor. The vapor then enters a condensing chamber surrounded by cold water to form a mixture, i.e. a condensate returning into a liquid phase from a gas phase. The mixture is then subjected to a separator to separate at least two portions including an upper portion and a lower portion. The upper portion refers to a layer floating on top of the mixture as it contains majority of volatile aroma compounds extracted from the plant material which are generally lighter in weight, thereby forming a concentrated essential oil. The lower portion refers a layer obtained at the bottom of the separator and below the floating layer. The lower portion has fewer amounts of aroma compounds than the upper portion and the compounds are generally heavier than that in the upper portion. The lower portion is considered as a water-based byproduct of the distillation process and is called hydrosol.

A hydrosol is considered to be less concentrated than an essential oil and may be applied to a subject without dilution, depending on its application. It is generally safe for use and causes less irritation than an essential oil. A hydrosol may be of varying grades and commercially available from vendors or companies around the world. The hydrosol used in preparing the composition of the present invention is of therapeutic grade, i.e. with undetectable or only trace amount of contaminants.

The composition includes a neroli hydrosol, a chamomile hydrosol and a rosemary hydrosol. It is believed that the combination of these hydrosols has unexpected and promising effect in increasing the dopamine level and exerting neuroprotective effect against oxidative damages. Therefore, it is useful in various applications especially when it is administered to an individual patient via oral route. The composition in an embodiment of the present invention is formulated as an oral composition including an oral pharmaceutical composition, a beverage, a food product including a food supplement, and the like.

The neroli hydrosol may have antibacterial and antifungal effects, as well as anti-inflammatory effect especially when applied on skin.

Typically, a chamomile hydrosol can be applied to promote calmness of a skin and has moisturizing and comforting effect.

Rosemary has been applied for years in flavoring in foods as well as in aromatherapy. Typically, a rosemary hydrosol can be applied topically to stimulate hair growth, skin recovery, and pain relief.

While the above hydrosols are generally used for topical applications to improve skin or hair condition, or used as room aromatics to reduce anxiety, the inventor surprisingly found that the oral administration of the above hydrosols in combination can achieve superior effects in increasing dopamine level, i.e. promoting psychological health of a subject, and protecting neuronal cells from oxidative stress. Without intending to be limited by theory, it is also believed that the invention can help to alleviate symptoms associated with a neurological disorder (e.g. a neurodegenerative disorder), a metabolic disorder, an autoimmune disorder, a gastrointestinal disorder, a cardiovascular disorder, a psychological disorder, or a mental disorder because of the increase in dopamine levels.

In an embodiment, the volume ratio of the neroli hydrosol to the chamomile hydrosol to the rosemary hydrosol is from about 1:1:1 to 10:1:1, such as about 1:1:1, about 2:1:1, about 3:1:1, about 4:1:1, about 5:1:1, about 6:1:1, about 7:1:1, about 8:1:1, about 9:1:1 or about 10:1:1. In a particular embodiment, the volume ratio of the neroli hydrosol to the chamomile hydrosol to the rosemary hydrosol is from about 6:1:1 to about 8:1:1, or about 6:1:1 or about 8:1:1.

It is appreciated that the composition may include one or more additional hydrosols, an additive, a carrier, and/or a therapeutic agent, depending on its use. In an embodiment where the composition further comprises an additional hydrosol, the volume ratio of the neroli hydrosol to the chamomile hydrosol to the rosemary hydrosol to the additional hydrosol may be from about 5:1:1:3, about 6:1:1:2, or about 7:1:1:1.

In addition to the hydrosols, the composition may include from about 0 vol.% to about 99.9 vol.% of water in a range from about 0.5 vol.% to about 99.5 vol.%, from about 5 vol.% to about 95 vol.%, from about 10 vol.% to about 90 vol.%, from about 20 vol.% to about 80% vol.%, from about 30 vol.% to about 70 vol.%, from about 40 vol.% to about 60 vol.%, about 50 vol.%, about 60 vol.%, about 70 vol.%, about 80 vol.%, or about 90% based on the total volume of the composition. Said volume of water refers to the water additionally added to the hydrosols, exclusive of the original water content in the hydrosols. In an embodiment, the volume ratio of the total hydrosol to water may be about 1:1 to about 1:20, about 1:1, about 1:2, about 1:5, about 1:9, about 1:10, about 1:15, about 1:19, or about 1:20. In another embodiment, the volume ratio of the total hydrosol to water may be from about 0.5:99.5 to about 5:95, in particular about 0.5:99:5, about 1:99, or about 2:98.

It is believed that the composition of the present invention may be effective in inducing dopamine production. The increase in dopamine level was observed by conducting an experiment with transgenic zebrafish as described in the examples. There are also clinical observations that the composition can help improve happiness and relieve stress and anxiety. Without intending to be limited by theory, it is thus believed that the composition is suitable for preparing a beverage, and an oral composition for promoting health including psychological and mental health of a user. It is also believed that the composition herein may have an antioxidant effect.

In an embodiment where the volume ratio of the total hydrosol to water is about 1:99, or about 0.5:99.5, the composition can be provided as a beverage for daily consumption. A user may find a composition with water easier to drink especially after exercise or under a warm or hot weather. The presence of water may, at the same time, help to improve blood circulation and facilitate the absorption of hydrosols.

The water used to prepare the composition can be a purified drinking water which may be selected from the group consisting of distilled water, deionized water, mineral water, reverse osmosis water (abbreviated as "RO water"), and a combination thereof. In an embodiment, the water is RO water. RO water refers to water purified by using partially permeable membrane under an applied pressure. RO water is generally devoid of minerals including heavy metals and has a reduced amount of suspended chemical and biological contaminants. It is thus clean and safe to drink and suitable for preparing an oral formulation. In an embodiment where the composition may be provided as a beverage, the composition contains at least about 10 vol.% to about 90 vol.% of RO water based on the total volume of the composition. In another embodiment, the composition contains from about 50 vol.% to about 95 vol.%, or about 75% to about 99 vol.% of RO water based on the total volume of the composition.

In an embodiment herein, the composition of the present invention contains from about 0.1 vol.% to about 10 vol.% of the neroli hydrosol, from about 0.01 vol.% to about 1 vol.% of the chamomile hydrosol, and from about 0.01 vol.% to about 1 vol.% of the rosemary hydrosol, based on the total volume of the composition. The composition may further comprise at least about 50 vol.% of water, or about 75.0 vol.% to about 99.9 vol.% water, based on the total volume of the composition.

In a particular embodiment, the composition contains about 0.4 vol.% of the neroli hydrosol, about 0.05 vol.% of the chamomile hydrosol, about 0.05 vol.% of the rosemary hydrosol, and about 99.5 vol.% of water. In an alternative embodiment, the composition contains about 0.3 vol.% of the neroli hydrosol, about 0.1 vol.% of an additional hydrosol, about 0.05 vol.% of the chamomile hydrosol, about 0.05 vol.% of the rosemary hydrosol, and about 99.5 vol.% of water. It would be appreciated that these embodiments containing a higher proportion of water are suitable to be formulated as beverages for daily needs. The continuous intake of this composition is useful to boost the immune system of an individual and improve his/her mood. This composition may also help to relieve stress. A kid or an adult can drink more than 100ml a day, e.g. 350ml a day and the composition can be provided as a packaged drink. The composition can also be provided to a patient as a part of remedy depending on the amount and frequency in administering the composition.

In another embodiment herein, the composition of the present invention contains at least about 40 vol.% of the neroli hydrosol, at least about 5 vol.% of the chamomile hydrosol, and at least about 5 vol.% of the rosemary hydrosol, based on the total volume of the composition. In particular, the composition contains from about 50 vol.% to about 80 vol.% of the neroli hydrosol, from about 5 vol.% to about 20 vol.% of the chamomile hydrosol, and from about 5 vol.% to about 20 vol.% of the rosemary hydrosol, and optionally water. For instance, the composition contains about 80 vol.% of the neroli hydrosol, about 10 vol.% of the chamomile hydrosol, and about 10 vol.% of the rosemary hydrosol, based on the total volume of the composition. These compositions contain a higher proportion of total hydrosol and are suitable to be formulated as a pharmaceutical composition or administered as a part of a remedy for therapeutic use.

The composition of the present invention, in particular the hydrosols thereof typically contain volatile aromatic compounds including at least α-terpineol, linalool, 1,8-cineole, camphor and coumarine. In an embodiment, α-terpineol, linalool, 1,8-cineole, camphor and coumarine are found in the neroli hydrosol, the chamomile hydrosol, and the rosemary hydrosol. In an embodiment, the composition includes at least about 50 mg/L of α-terpineol, at least about 20 mg/L of linalool, at least about 5 mg/L of 1,8-cineole, at least about 1 mg/L of camphor, and at least about 1 mg/L of coumarine.

In an embodiment, the composition may include
- from about 50 mg/L to about 120 mg/L, from about 70 mg/L to about 110 mg/L, or from about 80 mg/L to about 100 mg/L of α-terpineol;
- from about 20 mg/L to about 100 mg/L, from about 40 mg/L to about 80 mg/L, or from about 50 mg/L to about 70 mg/L of linalool;
- from about 5 mg/L to about 45 mg/L, from about 15 mg/L to about 35 mg/L, or from about 20 mg/L to about 30 mg/L of 1,8-cineole;
- from about 1 mg/L to about 30 mg/L, from about 10 mg/L to about 20 mg/L, or from about 15 mg/L to about 18 mg/L of camphor; and
- from about 1 mg/L to about 15 mg/L, from about 1 mg/L to about 7 mg/L, or from about 2 mg/L to about 5 mg/L of coumarine.

In one embodiment, the composition includes about 80 mg/L to about 90 mg/L of α-terpineol, about 50 mg/L to about 60 mg/L of linalool, about 25 mg/L to about 30 mg/L of 1,8-cineole, about 15 mg/L to about 18 mg/L of camphor, and about 2 mg/L to about 3 mg/L of coumarine particularly when the composition is provided as a beverage. Without intending to be limited by the theory, it is believed that the above components including α-terpineol, linalool, 1,8-cineole, camphor and coumarine in the composition act actively in promoting the health condition of a subject. It would be appreciated that the composition may further include, for example, linoleic acid, oleic acid, linalool oxides (fur.), methyl anthranilate, borneol, citronellol, palmitic acid, geraniol, and the like. The amount of the components as discussed above in the composition can be determined through suitable methods in the art. For instance, the components can be determined by conducting a gas chromatography (GC) in a research or commercial laboratory based on standard protocol and standard references.

Further, the composition has a pH of from about 4.0 to about 8.0, from about 5.0 to about 7.0, or from about 6.0 to about 6.5. Said pH value is suitable for a subject to drink.

As discussed above, the composition can be formulated as a pharmaceutical composition particularly an oral pharmaceutical composition. For ease of storage and administration, the composition may further comprise a preservative at a relatively low amount.

The subject herein is typically a mammal such as a rodent, a carnivore, or human. In an embodiment, the subject is human.

In another embodiment, the composition can be formulated as an eye drop and/or an eye rinse to rinse eyes of a user or to alleviate discomfort of eyes. For example, the composition formulated as an eye drop solution may include about 99.5 vol.% of pure water such as distilled water or reverse osmosis water, and about 0.5 vol.% of the total hydrosols, based on the total volume of the composition. The total hydrosols include the neroli hydrosol, the chamomile hydrosol and the rosemary hydrosol as described above and in particular in a volume ratio of about 8:1:1. In other words, the composition herein is also suitable for administration via ocular route. It is believed that the composition of the present invention can help alleviate irritations and pain in the eyes caused by dusts, undesired irritating gas particles, pollutants, and/or chemicals in contact with the eyes. It is also believed that the composition in the form of oral composition can also help to alleviate discomfort including pain and irritations as well as inflammation in the respiratory tract triggered by the above substances in the same occasion.

In a further embodiment, the composition can be provided as a beverage to supply nutrients and water to a subject. The beverage may be provided as a packaged drink in a bottle, a can or any suitable container. The beverage may be a nourishing and refreshing drink and at the same time mildly promote health of a subject. For example, the dopamine level may be elevated after drinking the beverage and therefore the subject may feel happier and relieved. It can help a user to calm down from stress and improve the sleep quality. In addition, due to the presence of tiny droplets/particles suspended in the beverage, e.g. droplets of essential oil or aromatic compounds present in the composition, shaking of the beverage can give a decent, sweet and fruity smell in which the user may find it more refreshing and soothing. It would be appreciated that the composition as described above can also be added to a food product.

Accordingly, the composition is suitable for the preparation of, respectively, an oral pharmaceutical composition, an eye drop, an eye rinse, a food product including a food supplement, and a beverage for promoting health of a subject.

In the second aspect, the present invention pertains to the composition for use in a method of treating, preventing and/or reducing the subject's risk of suffering from a disorder. The disorder may be selected from the group consisting of a neurological disorder (e.g. a neurodegenerative disorder), a metabolic disorder, an autoimmune disorder, a gastrointestinal disorder, a cardiovascular disorder, a psychological disorder, and a mental disorder. The method includes a step of administering an effective amount of the composition, as described above, to the subject. The subject is also as described above, in particular a rodent, a carnivore, or human.

In an embodiment where the subject is human, the composition is prepared as a pharmaceutical composition and administered to the subject in a volume of from about 1 ml to about 30 ml per day, or from about 3 ml to about 10 ml per day depending on the severity of the condition to be alleviated. In an embodiment, the composition is formulated in the form of an oral pharmaceutical composition and thus can be administered via oral route.

The composition is effective in treating, preventing and/or reducing the risk of suffering from a disorder associated with pain, muscle abnormality, headache, respiratory difficulties, depression, anxiety, fatigue, sleep difficulties, and/or dysphagia. It would be appreciated that the composition can be administered to the subject in combination with one or more additional therapeutic agents (e.g. including a pharmaceutical composition as described in Hong Kong Short Term Patent No. HK1221598 to Lai, published on 2 June 2017) depending on the condition of the subject.

The composition may be used in a method of alleviating symptoms associated with the disorder as described above by administering an effective amount of the composition of the present invention to the subject.

The examples set out below further illustrate the present invention. One skilled in the art understands that the embodiments described above as well as examples given below are not intended to be limiting.

### EXAMPLE 1

### Preparation of the composition

In the following examples, the composition of the present invention is prepared with the following ingredients:
- 3482.5 ml of reverse osmosis (RO) water;
- 14 ml of a neroli hydrosol (therapeutic grade);
- 1.75 ml of a chamomile hydrosol (therapeutic grade); and
- 1.75 ml of a rosemary hydrosol (therapeutic grade).
The total amount of the composition is 3500 ml and can be filled into ten bottles, each containing 350 ml of the composition. The pH is 6. All the hydrosols used in preparing the composition are of therapeutic grade and can be obtained through commercial suppliers.

A GC test was conducted to confirm the major components in the composition prepared according to the present invention. The GC test was conducted by a laboratory with standard testing conditions and procedures. Table 1 shows 10 components, among other components, in the composition.

**Table 1. GC results of 10 components in the composition.**

| **Component(s)** | **Amount** |
|---|---|
| α-terpineol | 84.1mg/L |
| Linalool | 57.8mg/L |
| 1,8-cineole | 25.5mg/L |
| Camphor | 16.0 mg/L |
| Coumarine | 2.3mg/L |
| Linoleic acid | 6.3 mg/L |
| Oleic acid | 8.9mg/L |
| Linalool oxides (fur.) | 9.7 mg/L |
| Methyl anthranilate | 11.4 mg/L |
| Borneol | 6.9 mg/L |

In order to determine whether the composition is safe for a user to drink or administer along with a therapeutic agent, several tests were conducted to evaluate the heavy metal content and microbial content.

A sample of the composition was analyzed to determine the content of heavy metal. The heavy metal test was conducted by a laboratory with standard testing conditions and procedures. The sample was digested by a method developed based on USEPA method 3005 prior to the determination of total metals. The results are as follow.

**Table 2. Results from heavy metal test on a sample of the composition.**

| Metals and Major Cations | Limit of reporting, LOR (µg/L) | Amount In the sample (µg/L) | Recommended guideline value (µg/L) |
|---|---|---|---|
| Antimony | 1 | <1 | 20 |
| Arsenic | 10 | <10 | 10 |
| Cadmium | 0.2 | <0.2 | 3 |
| Chromium | 1 | <1 | 50 |
| Lead | 1 | <1 | 10 |
| Mercury | 0.5 | <0.5 | 6 |
| Tin | 1 | <1 | - |

Based on the results, the levels of heavy metals in the composition did not exceed the guideline values as recommended by World Health Organization (WHO) for drinking water, see Guidelines for drinking water-quality issued by WHO.

Next, another sample of the composition was subjected to microbial test. The microbial test was also conducted by a laboratory with standard testing conditions and procedures. In the test, *Coliforms* bacteria including *E. Coli,* as well as *Staphylococcus aureus, Pseudomonas aeruginosa,* and *heterotrophs* were absent in the composition. They were not detected in the test.

Based on the test results, the composition does not have excess heavy metal and microbial contaminants and therefore it is safe to drink and to be provided as a beverage including drinking water for consumers and individuals.

### Example 2

### Effect in dopamine production

Dopamine is a key reward neurotransmitter which has an important role in the brain reward system. It can regulate normal behavior, motivation and emotion, and affects movement, memory and focus. In general, the increase in dopamine will result in a better mood and therefore it is useful in regulating the emotion of a subject.

Tyrosine hydroxylase (TH) is the rate limiting enzyme in dopamine synthesis. The expression of TH can reflect the level of dopamine in a subject. A test was thus conducted to determine the effect of the composition of the present invention in dopamine production by using transfected zebrafish. The zebrafish was particularly transfected with green fluorescent protein (GFP) to indicate the presence of TH.

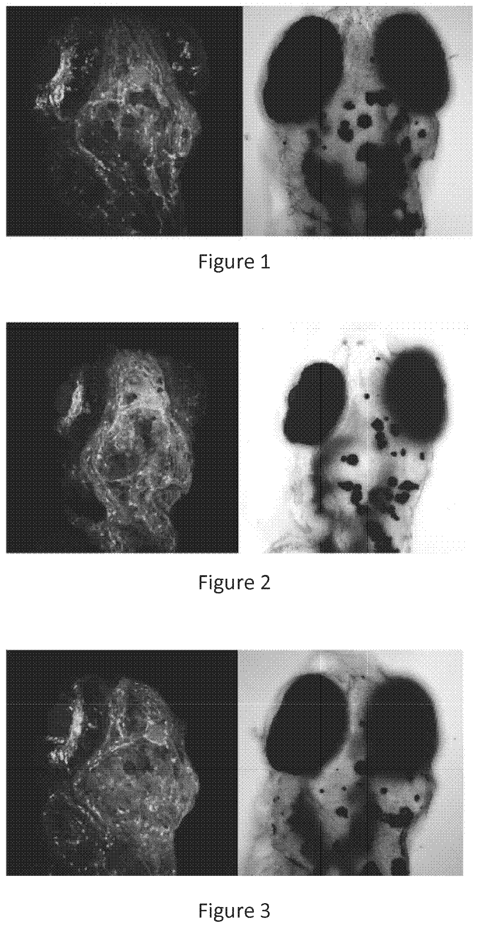

As shown in Figure 2, TH-GFP expression increased after treating the transfected zebrafish with the composition of the present invention, i.e. the composition as prepared in Example 1, compared to the control group as shown in Figure 1. Another transfected zebrafish was treated with a diluted composition, i.e. the composition was diluted 1/10. The results as shown in Figure 3 reveals that the diluted composition has less effect on TH-GFP expression compared to the non-diluted one. The effect of the composition may be on a dose-dependent manner.

Based on the results, the composition of the present invention can induce the level of TH and thereby increasing the production of dopamine. A subject being administered or treated with the composition may have a better mood, and exhibit improved motion or emotional reactions.

### Example 3

### Preventive protective effect in neurons

To investigate whether the composition of the present invention has any protective effect on neurons under oxidative stress, a neurite outgrown assay was performed.

It is assumed that the protective effect of the composition can be reflected via the changes in the length of neurites. When the average length of neurites increases or remains substantially unchanged after oxidative challenge, the composition is considered to exert a protective effect on neurites, e.g. protect the neurons from oxidative stress. In other words, when the average length of neurites decreases after oxidative challenge, the composition fails to exert a protective effect.

In the experiment, SH-SY5Y cells, i.e. human neuroblastoma cell line (ATCC^{®} CRL-2266^{™}), were used. Before subjecting the cells under oxidative stress condition, an experiment was conducted to evaluate the effect of the composition of the present invention on the cells, e.g. to determine a suitable range of working concentration for the cells. The composition was prepared in different concentrations by diluting it with a plain cell culture medium (DMEM/F12). First of all, SH-SY5Y cells were treated with the composition, the composition of Example 1, at the concentration of 1%, 0.1%, 0.01% or 0.001% according to the following groups for 24 or 48 hours.

**Table 3. Concentration of the composition in the control group and four treatment groups.**

| **Group** | **Volume Conc. of the composition** |
|---|---|
| Control | 0% |
| Group A | 1% |
| Group B | 0.1% |
| Group C | 0.01% |
| Group D | 0.001% |

Upon the specified time points, the treated cells were fixed and incubated in PBS for subsequent measurement. The length of neurites in SH-SY5Y cells was measured by Motic Image Plus 2.0 software. Thirty cells were counted in each group. The average length of neurites (AU) is plotted against the control group and treatment groups for comparison. The statistical significance of the experimental data of multiple groups is determined by Student's paired t-test for two sets of samples and the results are expressed by mean ± standard deviation. In the test, p <0.05 was considered as statistically significant.

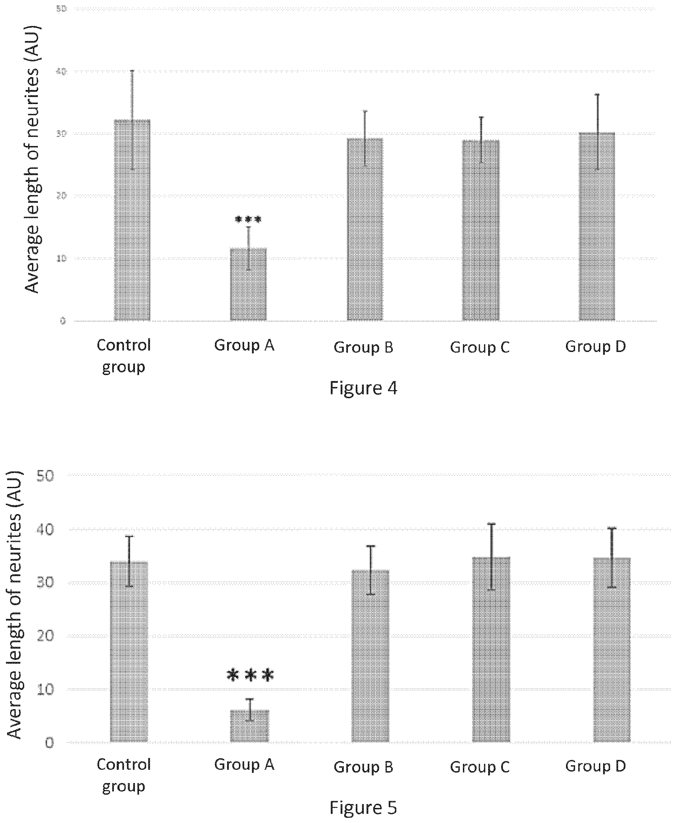

Figure 4 shows the average length of neurites after treating the cells with the composition for 24 hours. Groups B, C and D, i.e. treated with 0.1%, 0.01% or 0.001 % of the composition, have no significant changes in the length of the neurites, similar to the control group. However, there is a significant reduction in Group A, showing that 1% of the composition may not be suitable for SH-SY5Y cells. Similarly, Figure 5 shows the average length of neurites after treating the cells with the composition for 48 hours. The results show that the incubation time does not significant alter the effect of the composition on the cells. Groups B, C and D have no obvious effect on the cells while Group A results in a reduction in length of the neurites. Accordingly, it is believed that the composition may work better at a concentration of 0.1%, 0.01% and 0.001 % for SH-SY5Y cells.

In the next experiment, the SH-SY5Y cells were pre-treated with the composition at different concentrations, followed by oxidative stress challenge induced by hydrogen peroxide. The treated cells were then incubated at 37°C with 5% CO₂ in humidified incubator for 24 and 48 hours. After incubation, the cells were challenged by 500mM hydrogen peroxide (H₂O₂) for 3 hours at 37°C so as to induce oxidative stress. Upon the specified time points, the treated cells were fixed and incubated in PBS for measurement. The length of neurites in SH-SY5Y cells was measured by Motic Image Plus 2.0 software. Thirty cells were counted in each group. The average length of neurites (AU) is plotted against the control group and treatment groups for comparison. The statistical significance of the experimental data of multiple groups was determined by Student's paired t-test for two sets of samples and the results are expressed by mean ± standard deviation. In the test, p <0.05 was considered as statistically significant, in which p ≤ 0.05 *, p ≤ 0.01 **, p ≤ 0.001 ***.

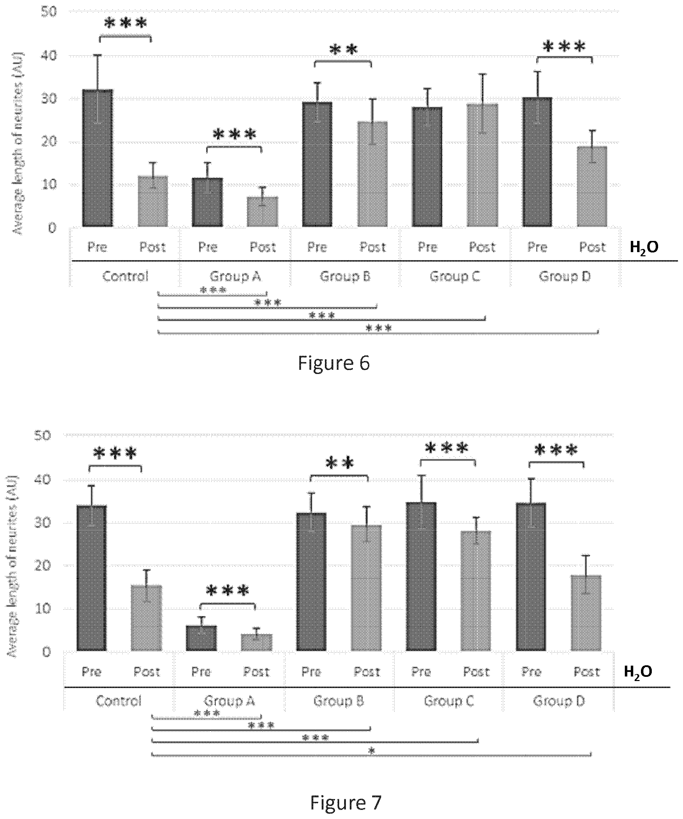

Figure 6 shows the average length of neurites of SH-SY5Y cells in different groups before and after oxidative stress challenge, in which the cells were pretreated for 24 hours before the challenge.

There are two results for each group, i.e. "pre" and "post", indicating the results obtained before H₂O₂ challenge and after the challenge for each group. The neurites in the control group exhibit significant reduction by more than 50% in the average length after the challenge, which means the cells were under significant stress. For the cells in Groups B, C and D, there are no significant changes in the average length of neurites after incubation with the composition prior to the challenge. Although the average length of neurites reduces or almost remains unchanged after the challenge, the length is still higher than that in the control group. Accordingly, the composition is found to have protective effect on neurons at a volume concentration of 0.1 %, 0.01% and 0.001%, compared to the control group.

For the cells in Group A, the results suggest that 1% of the composition is not suitable for incubating SH-SY5Y cells because it might induce obvious oxidative stress in the cells. Therefore, a lower concentration of the composition is preferred.

Figure 7 shows the average length of neurites of SH-SY5Y cells in different groups before and after oxidative stress challenge, in which the cells were pretreated for 48 hours before the challenge.

Similar to the 24-hour pretreatment, the neurites in the control group exhibit significant reduction in the length after 48-pretreatment followed by the challenge. For the cells in Groups B and C, i.e. cells treated with 0.1 and 0.01% of the composition before the challenge are found to have longer average length (p-value < 0.001) than that in the control group. Even at the concentration of 0.001%, the average length of neurites is still found moderately longer (p-value < 0.05) than that in the control group. Accordingly, the composition is found to have protective effect on neurons at a relatively lower concentration.

The results for the cells in Group A reflect that 1% of the composition is not suitable for incubating SH-SY5Y cells because it might induce obvious oxidative stress in the cells.

Based on the above results, it is found that the cellular damage induced by H₂O₂ challenge is lessened if the cells were pre-treated with the composition of the present invention. Hence, the results imply that the composition can ameliorate the oxidative damage in SH-SY5Y cells challenged by H₂O₂.

### Example 4

### Therapeutic effect in neurons

Another neurite outgrowth assay was performed to determine whether the composition can stimulate recovery and growth in neural cells which were already damaged by oxidants. Similar to Example 3, neurite length was used to determine the neuroprotective effect under oxidative stress. SH-SY5Y cells were used in the assay and exposed to H₂O₂ for 5 hours before addition and incubation of the composition of the present invention.

Interestingly, it was found that neurites reduced in length after 12 hours and then grew again in another 12 hours, although the neurite length still could not be compared to its initial length.

First, SH-SY5Y cells, after being exposed to the H₂O₂, were divided into 4 groups, i.e. one control group and three treatment groups. The composition was prepared in different concentrations as follows, while the control group referred to the cell sample without treatment with the composition.

**Table 4. Concentration of the composition in the control group and three treatment groups.**

| **Group** | **Volume Conc. of the composition** | **Dilution** |
|---|---|---|
| Control | 0% | NA |
| Group I | 10% | 1/10 dilution |
| Group II | 1% | 1/100 dilution |
| Group III | 0.1% | 1/1000 dilution |

After addition of the composition, the cells were incubated for 24 hours and the length of the neurites was measured at 12th hour and 24th hour.

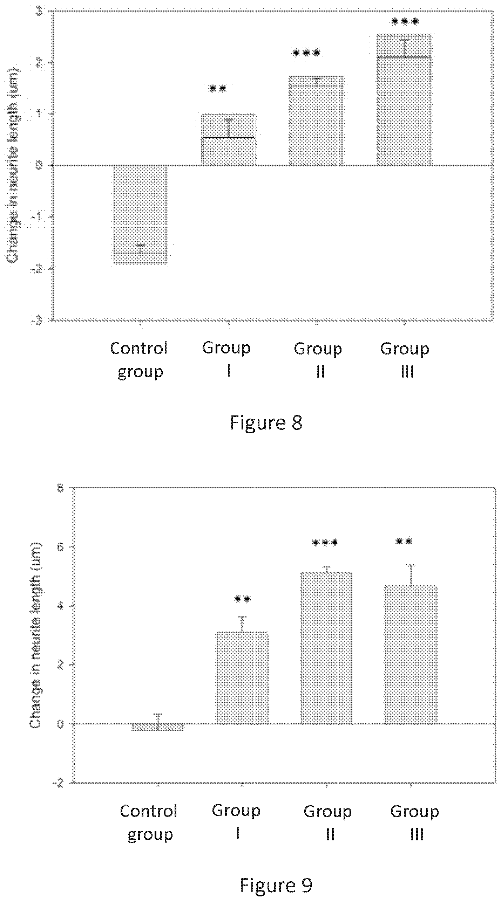

Figure 8 shows the changes in the length of neurites after 12-hour treatment. The results show that the composition can alleviate the effect caused by H₂O₂ challenge, in particular the cells treated with the composition had an increased length in neurites. Also, the lower the concentration of the composition, the better neuroprotective effect to elongate the neurites against the oxidative stress.

Figure 9 shows the changes in the length of neurites after 24-hour treatment. Although the length of the neurites in the control group almost restored to their initial lengths after the additional 12-hour incubation, treatment groups still exhibit remarkable increase in the length compared to the control group. Accordingly, the composition has neuroprotective effect to cells under oxidative stress.

### Example 5

### Therapeutic effect in epilepsy

In order to study whether the composition of the present invention (i.e. the composition in Example 1) has any inhibitory effect on epilepsy (EP), various experiments were performed on experimental epilepsy-affected mice.

As known to those skilled in the art, pentylenetetrazole (PTZ) is a powerful seizure-inducing agent, and its epileptic model is considered to be one of the ideal models for systemic tonic seizures. PTZ itself has no special neurotoxicity, so it is widely used in the study of epilepsy pathogenesis and the screening of anti-epileptic drugs. When PTZ induces epilepsy in mice, its seizures are characterized by clonic seizures in the initial stage, followed by systemic tonic seizures soon afterwards. PTZ is a central nervous system stimulant that acts on GABA-GABAA receptor system. It promotes the enhancement of the excitatory synaptic facilitation and reduces the release of GABA. This in turn reduces the inhibitory ability, increases the sensitivity to convulsions, and increases the excitability of the central nervous system, thereby inducing EP. According to its seizure characteristics, in this experiment, these two seizure forms were used as indicators and combined with the current Racine scale to analyze their epileptic behavior.

Carbamazepine (CBZ) was also used as one of the comparison groups in this experiment. CBZ is a known anticonvulsant that works by reducing nerve impulses that cause seizures and neuralgia. It has a stabilizing effect and can reduce the permeability of nerve cell membranes to Na+ and Ca2+, thereby reducing cell excitability. It prolongs the refractory period and may also enhance the synaptic transmission ability of GABA.

First, 72 healthy male ICR mice were randomly divided into 6 groups of 12 mice: blank control group (0.5 vol% sodium carboxymethyl cellulose solution), PTZ model group (0.5 vol% sodium carboxymethyl cellulose solution), carbamazepine group (80 mg/kg of carbamazepine), and high-, medium-, and low-dose groups of the composition of the present invention. The mice in each group were given intragastric administration daily, for 7 consecutive days. 30 minutes after the last administration, except for the blank control group, the mice in the other groups were injected intraperitoneally with 80 mg/kg of PTZ to induce convulsions in the mice. Each mouse was placed in a separate cage for observation for 30 minutes, and behavioral changes such as stages of seizure, duration, latent period and mortality rate of the mice were recorded.

The Racine scale (see: https://en.wikipedia.org/wiki/Racine_stages) was used to determine the level of epilepsy in mice:
Stage 0: No response;
Stage I: Wet dog shakes, facial twitches, clonus, tremors (such as eye blinking, moustache movements, rhythmic chewing, etc.);
Stage II: Stage I plus rhythmic nodding;
Stage III: Stage II plus forelimb clonus, but no rearing;
Stage IV: Rearing and oblique bilateral falling, or rearing with forelimb clonus; and
Stage V: Imbalance, tipping, twitching of limbs, generalized clonus, generalized tonic seizures, falling, rolling, and loss of postural control.

First, experiments were conducted to determine the effect of the composition of the present invention on the seizure behavior of PTZ-induced epileptic mice. After the intraperitoneal injection of PTZ in mice, the mice showed symptoms of EP. It can be seen from Table 5 that, compared with the model group, the composition of the present invention has a significant improved effect on the behavior of epileptic mice. In this experiment, the high-, medium-, and low-dose groups of the composition had statistical differences (p<0.05), in which when compared with the blank group, #p<0.05; and when compared with the model group, *p<0.05.

**Table 5. Effect on the seizure behavior of PTZ-induced epileptic mice (n=12).**

| **Group** | **Stage 0** | **Stage I** | **Stage II** | **Stage III** | **Stage IV** | **Stage V** |
|---|---|---|---|---|---|---|
| Blank control group | 0 | 0 | 0 | 0 | 0 | 0 |
| PTZ model group | 0 | 0 | 0 | 1 | 1 | 10# |
| Carbamazepine group | 1 | 3 | 3 | 2 | 2 | 1* |
| High-dose group | 0 | 3 | 2 | 2 | 3 | 2* |
| Medium-dose group | 0 | 2 | 4 | 3 | 2 | 1* |
| Low-dose group | 0 | 1 | 2 | 2 | 4 | 3* |

Then, experiments were conducted to determine the effect of the composition on PTZ-induced epileptic mice. Studies have shown that PTZ can induce convulsions by reducing the content of GABA in the brain tissues of the mice. Therefore, intraperitoneal injection of 80mg/kg PTZ in mice can induce the probability of convulsions. Table 6 shows the effect of the composition on convulsions in PTZ-induced epileptic mice, in which when compared with the blank group, #p<0.05; and when compared with the model group, *p<0.05; **p<0.01. Compared with the blank control group, all the mice in the PTZ model group had convulsions, indicating that the modelling was successful. Compared with the PTZ model group, the high-dose group can significantly prolong the latent period of convulsions in PTZ-induced epileptic mic, shorten the duration of convulsions, reduce the intensity of convulsions, and reduce the mortality rates.

**Table 6. Effect on convulsions in PTZ-induced epileptic mice (n=12).**

| **Group** | **Number of convulsions** | **Latent period of convulsions/s** | **Duration of convulsions/s** | **Morality rates/%** |
|---|---|---|---|---|
| Blank control group | 0 | / | / | 0 |
| PTZ model group | 12 | 76.6±40.3# | 234.2±34.8# | 100 |
| Carbamazepine group | 8 | 365.2±25.6** | 165.3±32.5* | 16.7 |
| High-dose group | 8 | 247.4±56.2** | 132.5±26.4** | 25.0 |
| Medium-dose group | 8 | 243.2±34.8** | 160.3±23.1 * | 33.3 |
| Low-dose group | 8 | 146.1±45.6 | 194.1±46.1 | 75.0 |

In addition, experiments were also conducted to determine the effect of the composition on the number of seizures in PTZ-induced epileptic mice. Table 7 shows the results of the experiments, in which when compared with the blank group, #p<0.05; when compared with the model group, *p<0.05; **p<0.01. Compared with the model group, the carbamazepine group and the high-dose group can significantly reduce the number of seizures in stage IV EP mice (p<0.05). The high-dose group can significantly reduce the number of seizures in stages IV and V EP mice (p<0.01). The medium-dose group can significantly reduce the number of seizures of stage V EP mice (p<0.01). The low-dose group can significantly reduce the number of seizures of stage V EP mice (p<0.05).

**Table 7. Effect on the number of seizures in PTZ-induced epileptic mice (n=12).**

| **Group** | **Stage I** | **Stage II** | **Stage III** | **Stage IV** | **Stage V** |
|---|---|---|---|---|---|
| Blank control group | 0 | 0 | 0 | 0 | 0 |
| PTZ model group | 1.8±3.5# | 1.6±1.2# | 3.2±1.8# | 4.8±2.9# | 7.3±2.5# |
| Carbamazepine group | 0.9±0.4 | 1.4±0.8 | 1.5±0.8* | 2.1±1.8* | 6.3±2.1 |
| High-dose group | 4.8±2.5** | 3.9±0.8** | 1.6±2.0 | 1.9±0.6** | 3.1±1.9** |
| Medium-dose group | 2.6±0.9 | 3.9±1.3** | 5.9±0.7** | 4.2±2.8 | 4.1±0.8** |
| Low-dose group | 2.2±1.1 | 2.0±0.9 | 2.9±2.1 | 4.6±1.8 | 3.8±3.1 * |

After completing the behavioral observation determined by the above relevant indicators, experiments were conducted to study the effect of the composition on the contents of NO, Na-K-ATP, Asp, and 5-HT in the brain of convulsive mice. After the behavioral observation was over, the mice were immediately executed, and the brain tissues and the required organs were quickly taken out, weighed, and then the brain tissues were placed in liquid nitrogen and stored in a refrigerator at -80°C. Blood stains on the surface were washed off with pre-cooled saline before measurement. The tissues were then weighed and PBS was added in a volume ratio of 1:9 to fully homogenize the brain tissues. The homogenate was centrifuged at 3000r/min for 20 minutes. The supernatant was collected as samples for subsequent ELISA test to detect the content of GABA, Glu, NO, Na⁺-K⁺-ATP, Asp, 5-HT, etc.

As shown in Table 8, compared with the blank control group, the NO concentration in the brain tissues of the PTZ model group mice was significantly increased (p<0.01). Compared with the PTZ model group, the high- and medium- dose groups can both significantly (p<0.05) reduce the concentration of NO in the brain of convulsive mice, but the effect is slightly worse than that of carbamazepine. The low-dose group has no significant effect (p>0.05).

**Table 8. Effect on the contents of NO, Na-K-ATP, Asp, and 5-HT in the brain of convulsive mice (n=12).**

| **Group** | **NO(µmol/L)** | **Na-K-ATP(ng/L)** | **Asp(pg/mL)** | **5-HT(ng/L)** |
|---|---|---|---|---|
| Blank control group | 32.5±2.3 | 180.1±32.3 | 40.3±2.5 | 12.3±1.2 |
| PTZ model group | 43.2±3.1## | 132.4±4.5# | 52.3±5.6## | 7.4±0.9# |
| Carbamazepine group | 26.4±2.5** | 192.1±5.7** | 42.1±1.2** | 14.6±2.6** |
| High-dose group | 29.2±1.1** | 182.0±8.9** | 41.3±0.9** | 13.2±4.5** |
| Medium-dose group | 33.4±2.4 | 176.7±46.2* | 50.2±3.1 | 12.6±4.5** |
| Low-dose group | 33.2±1.9 | 152.6±7.8 | 49.3±4.6 | 11.9±2.2* |

Based on the above experimental results, it was found that the composition of the present invention can effectively resist PTZ-induced epileptic seizures in mice, reduce the excitability, reduce number of seizures and their degree, and have significant promising anti-epileptic effects. There is also a dose-dependent effect.

It is well known that the contents of GABA, Glu, Asp and 5-HT in the brain is critical to the occurrence of epilepsy. Glu and Asp are excitatory amino acids, and GABA and 5-HT are inhibitory amino acids. Excessive Glu content can destroy the neurotoxins of the central neurons, thus producing excitement and causing brain damage.

In this example, the high and medium doses of the composition can significantly adjust the GABA concentration in the brain tissues of PTZ-induced epileptic mice, thereby reducing the degree of brain tissue damage. GABA receptors are divided into two types: GABAa and GABAb. GABA receptors are expressed on Glu-ergic synaptic terminals and GABA-ergic synaptic terminals. GABAb receptor agonists can activate these receptors and inhibit GABA-ergic and Glu-ergic synapses, thereby inhibiting the release of GABA and Glu and exerting a central inhibitory effect. Experiments have shown that both high and medium doses of the composition can significantly alleviate the increase in Glu concentration in the brain tissues of mice caused by N-methyl-D-aspartic acid (NMDA), increase 5-HT levels, and reduce Asp levels. It also effectively reduces the stage of epileptic seizures, prolongs the latent period of convulsions, shortens the time of epileptic seizures, and has a promising anti-epileptic effect.

### Example 6

### Therapeutic effect in diabetic nephropathy

In order to study whether the composition of the present invention has any inhibitory effect on diabetic nephropathy (DN), various experiments were performed on experimental rats.

First, under normal eating and drinking conditions, each rat was first intraperitoneally injected with 0.5 mL of complete freund's adjuvant (CFA), and then intraperitoneally injected with streptozotocin (STZ) solution (a dosage of 30 mg/kg) the next day. The above steps were repeated once a week for two consecutive weeks. 72 hours after the second injection of STZ, diabetic (DM) rats with fasting blood glucose values between 16.8-25 mmol/L were selected and continued to be raised for a total of 30 days. The preliminary test results showed that on the 30th day of the experiment, compared with the normal group of rats, the urine microalbuminuria (MALB) of the DM rats was significantly increased, and the kidney weight/body weight ratio and the glomerular filtration rate (GFR) increased, indicating that the DM rats had developed kidney disease, proving the success of the DN models. The model group and genetically modified (GM) group shown in Table 9 used the above-mentioned DN models. Rats in the GM group were given intragastric administration (i.e. the composition of the present invention) at 8 o'clock in the morning on the day of grouping, and the normal control group and the model control group were given physiological saline, each with a volume of 1 mL/100 g body weight and once daily for four consecutive weeks.

In order to measure the serum angiotensin II (Ang II), the rats were fasted for 12 hours after the last administration, and 6 mL of blood was collected from the femoral vein after anesthesia and placed in ethylenediaminetetraacetic acid (EDTA), heparin anticoagulation test tubes, then centrifuged at 4°C 3000r/min to separate the plasma, and stored at a low temperature for testing.

As shown in Table 9 (in which when compared with the normal group, *p<0.01; when compared with the model group, #p<0.01), compared with the normal control group, Ang II of the model group increased. After GM treatment, Ang II decreased, and there was a significant difference compared with the model group (p<0.01).

**Table 9. Effect on Ang II of DN rats.**

| **Active drug group** | **Concentration** | **Ang II (pg/ml)** |
|---|---|---|
| Normal control active drug group | / | 96.42±9.43 |
| Model group | / | 245.91±13.34* |
| GM group | 0.4ml/200g | 164.22±16.13# |

In addition, experiments were also conducted to determine the effect of the composition of the present invention on the kidney weight/body weight ratio and GFR. One hour after the last administration, both kidneys were taken out and weighed on an electronic balance to calculate the kidney weight/body weight ratio. Also, one hour after the last administration, the rats were anesthetized with sodium pentobarbital, blood was collected from the abdominal aorta, and the serum was separated by centrifugation. On the day before the rats were sacrificed, the urine of each group of rats within 24 hours was collected by the metabolic cage method. A colorimetric method was used to determine the blood and urine creatinine contents. According to blood and urine creatinine values and urine output per minute, GFR is calculated, where GFR = urine creatinine × urine output per minute / blood creatinine.

As shown in Table 10 (in which when compared with the normal group, *p<0.01; when compared with the model group, #p<0.01), compared with the normal control group, the kidney weight/body weight ratio and GFR increased in the model group of rats. After GM treatment, the kidney weight/body weight ratio and GFR decreased, which were significantly different from the model group (p<0.01).

**Table 10. Effect on kidney weight/body weight ratio and glomerular filtration rate of DN rats.**

| **Group** | **Concentration** | **Kidney weight/body weight (g/Kg)** | **GFR (ml/min)** |
|---|---|---|---|
| Normal control group | / | 6.56±1.58 | 0.64±0.4 |
| Model group | / | 10.16±1.28* | 18.92±7.52* |
| GM group | 0.4ml/200g | 8.18±2.01# | 9.61±4.61# |

It would be appreciated by persons skilled in the art that numerous variations and/or modifications may be made to the the specific embodiments described. The present embodiments are, therefore, to be considered in all respects as illustrative and not restrictive.

## Claims

1. A composition comprising a neroli hydrosol, a chamomile hydrosol and a rosemary hydrosol.

2. The composition of claim 1, wherein a volume ratio of the neroli hydrosol to the chamomile hydrosol to the rosemary hydrosol is from about 1:1:1 to about 10:1:1.

3. The composition of claim 1 or claim 2, which comprises an additional therapeutic agent.

4. The composition of any one preceding claim, wherein the composition comprises from about 0.1 vol.% to about 10 vol.% of the neroli hydrosol, from about 0.01 vol.% to about 1 vol.% of the chamomile hydrosol, and from about 0.01 vol.% to about 1 vol.% of the rosemary hydrosol, based on the total volume of the composition; and wherein the composition optionally comprises at least 50 vol.% of water based on the total volume of the composition; or from about 75.0 vol.% to about 99.9 vol.% of water based on the total volume of the composition.

5. The composition of any one preceding claim, wherein the composition comprises:
- from about 50 mg/L to about 120 mg/L of α-terpineol; or from about 70 mg/L to about 110 mg/L of α-terpineol; or from about 80 mg/L to about 100 mg/L of α-terpineol; or from about 80 mg/L to about 90 mg/L of α-terpineol;
- from about 20 mg/L to about 100 mg/L of linalool; or from about 40 mg/L to about 80 mg/L of linalool; or from about 50 mg/L to about 70 mg/L of linalool; or from about 50 mg/L to about 60 mg/L of linalool;
- from about 5 mg/L to about 45 mg/L of 1,8-cineole; or from about 15 mg/L to about 35 mg/L of 1,8-cineole; or from about 20 mg/L to about 30 mg/L of 1,8-cineole; or from about 25 mg/L to about 30 mg/L of 1,8-cineole;
- from about 1 mg/L to about 30 mg/L of camphor; or from about 10 mg/L to about 20 mg/L of camphor; or from about 15 mg/L to about 18 mg/L of camphor; or about 15 mg/L to about 18 mg/L of camphor; and
- from about 1 mg/L to about 15 mg/L of coumarine; from about 1 mg/L to about 7 mg/L of coumarine; or from about 2 mg/L to about 5 mg/L of coumarine; or from about 2 mg/L to about 3 mg/L of coumarine.

6. The composition of any one preceding claim having a pH of from about 5 to about 7.

7. The composition of any one preceding claim, wherein the composition is selected form the group comprising an oral composition, a pharmaceutical composition or a beverage composition; or an oral composition; or a pharmaceutical composition; or a beverage composition.

8. The composition of any one preceding claim for use in treatment of or reducing the risk of suffering from a disorder.

9. The composition of any one of claims 1 to 7 for use in treatment of or reducing the risk of suffering from a disorder, wherein the disorder is selected from a group consisting of a neurodegenerative disorder caused by oxidative stress, epilepsy and diabetic nephropathy.

## Patentansprüche

1. Zusammensetzung, umfassend ein Nerolihydrolat, ein Kamillenhydrolat und ein Rosmarinhydrolat.

2. Zusammensetzung nach Anspruch 1, wobei ein Volumenverhältnis von dem Nerolihydrolat zu dem Kamillenhydrolat zu dem Rosmarinhydrolat von etwa 1:1:1 bis etwa 10:1:1 beträgt.

3. Zusammensetzung nach Anspruch 1 oder Anspruch 2, die ein zusätzliches therapeutisches Mittel umfasst.

4. Zusammensetzung nach einem vorstehenden Anspruch, wobei die Zusammensetzung, bezogen auf das Gesamtvolumen der Zusammensetzung, von etwa 0,1 Vol.-% bis etwa 10 Vol.-% das Nerolihydrolat, von etwa 0,01 Vol.-% bis etwa 1 Vol.-% das Kamillenhydrolat und von etwa 0,01 Vol.-% bis etwa 1 Vol.-% das Rosmarinhydrolat umfasst; und wobei die Zusammensetzung gegebenenfalls mindestens 50 Vol.-% Wasser, bezogen auf das Gesamtvolumen der Zusammensetzung; oder von etwa 75,0 Vol.-% bis etwa 99,9 Vol.-% Wasser, bezogen auf das Gesamtvolumen der Zusammensetzung, umfasst.

5. Zusammensetzung nach einem vorstehenden Anspruch, wobei die Zusammensetzung Folgendes umfasst:
- von etwa 50 mg/l bis etwa 120 mg/l α-Terpineol; oder von etwa 70 mg/l bis etwa 110 mg/l α-Terpineol; oder von etwa 80 mg/l bis etwa 100 mg/l α-Terpineol; oder von etwa 80 mg/l bis etwa 90 mg/l α-Terpineol;
- von etwa 20 mg/l bis etwa 100 mg/l Linalool; oder von etwa 40 mg/l bis etwa 80 mg/l Linalool; oder von etwa 50 mg/l bis etwa 70 mg/l Linalool; oder von etwa 50 mg/l bis etwa 60 mg/l Linalool;
- von etwa 5 mg/l bis etwa 45 mg/l 1,8-Cineol; oder von etwa 15 mg/l bis etwa 35 mg/l 1,8-Cineol; oder von etwa 20 mg/l bis etwa 30 mg/l 1,8-Cineol; oder von etwa 25 mg/l bis etwa 30 mg/l 1,8-Cineol;
- von etwa 1 mg/l bis etwa 30 mg/l Kampfer; oder von etwa 10 mg/l bis etwa 20 mg/l Kampfer; oder von etwa 15 mg/l bis etwa 18 mg/l Kampfer; oder etwa 15 mg/l bis etwa 18 mg/l Kampfer; und
- von etwa 1 mg/l bis etwa 15 mg/l Cumarin; von etwa 1 mg/l bis etwa 7 mg/l Cumarin; oder von etwa 2 mg/l bis etwa 5 mg/l Cumarin; oder von etwa 2 mg/l bis etwa 3 mg/l Cumarin.

6. Zusammensetzung nach einem vorstehenden Anspruch, die einen pH-Wert von etwa 5 bis etwa 7 aufweist.

7. Zusammensetzung nach einem vorstehenden Anspruch, wobei die Zusammensetzung ausgewählt ist aus der Gruppe umfassend eine orale Zusammensetzung, eine pharmazeutische Zusammensetzung oder eine Getränkezusammensetzung; oder eine orale Zusammensetzung; oder eine pharmazeutische Zusammensetzung; oder eine Getränkezusammensetzung.

8. Zusammensetzung nach einem vorstehenden Anspruch zur Verwendung bei der Behandlung von oder zum Verringern des Risikos des Leidens an einer Störung.

9. Zusammensetzung nach einem der Ansprüche 1 bis 7 zur Verwendung bei der Behandlung von oder zum Verringern des Risikos des Leidens an einer Störung, wobei die Störung ausgewählt ist aus einer Gruppe bestehend aus einer neurodegenerativen Störung, die durch oxidativen Stress verursacht wird, Epilepsie und diabetischer Nephropathie.

## Revendications

1. Composition comprenant un hydrolat de néroli, un hydrolat de camomille et un hydrolat de romarin.

2. Composition selon la revendication 1, dans laquelle le rapport volumique de l'hydrolat de néroli à l'hydrolat de camomille à l'hydrolat de romarin est d'environ 1:1:1 à environ 10:1:1.

3. Composition selon la revendication 1 ou la revendication 2, qui comprend un agent thérapeutique supplémentaire.

4. Composition selon une quelconque revendication précédente, dans laquelle la composition comprend d'environ 0,1 % à environ 10 % en volume d'hydrolat de néroli, d'environ 0,01 % à environ 1 % en volume d'hydrolat de camomille et d'environ 0,01 % à environ 1 % en volume d'hydrolat de romarin, par rapport au volume total de la composition ; et dans laquelle la composition comprend éventuellement au moins 50 % en volume d'eau par rapport au volume total de la composition ; ou d'environ 75,0 % à environ 99,9 % en volume d'eau par rapport au volume total de la composition.

5. Composition selon une quelconque revendication précédente, dans laquelle la composition comprend :
- d'environ 50 mg/L à environ 120 mg/L d'α-terpinéol ; ou d'environ 70 mg/L à environ 110 mg/L d'α-terpinéol ; ou d'environ 80 mg/L à environ 100 mg/L d'α-terpinéol ; ou d'environ 80 mg/L à environ 90 mg/L d'α-terpinéol ;
- d'environ 20 mg/L à environ 100 mg/L de linalol ; ou d'environ 40 mg/L à 80 mg/L de linalol ; ou d'environ 50 mg/L à environ 70 mg/L de linalol ; ou d'environ 50 mg/L à environ 60 mg/L de linalol ;
- d'environ 5 mg/L à environ 45 mg/L de 1,8-cinéole ; ou d'environ 15 mg/L jusqu'à environ 35 mg/L de 1,8-cinéole ; ou d'environ 20 mg/L à environ 30 mg/L de 1,8-cinéole ; ou d'environ 25 mg/L à environ 30 mg/L de 1,8-cinéole ;
- d'environ 1 mg/L à environ 30 mg/L de camphre ; ou d'environ 10 mg/L à environ 20 mg/L de camphre ; ou d'environ 15 mg/L à environ 18 mg/L de camphre ; ou d'environ 15 mg/L à environ 18 mg/L de camphre ; et
- d'environ 1 mg/L à environ 15 mg/L de coumarin ; d'environ 1 mg/L à environ 7 mg/L de coumarin ; ou d'environ 2 mg/L à environ 5 mg/L de coumarin ; ou d'environ 2 mg/L à environ 3 mg/L de coumarin.

6. Composition selon une quelconque revendication précédente présentant un pH d'environ 5 à environ 7.

7. Composition selon une quelconque revendication précédente, dans laquelle la composition est choisie dans le groupe comprenant une composition orale, une composition pharmaceutique ou une composition de boisson ; ou une composition orale ; ou une composition pharmaceutique ; ou une composition de boisson.

8. Composition selon une quelconque revendication précédente pour une utilisation dans le traitement ou la réduction du risque de souffrir d'un trouble.

9. Composition selon l'une quelconque des revendications 1 à 7 pour une utilisation dans le traitement ou la réduction du risque de souffrir d'un trouble, dans laquelle le trouble est sélectionné dans un groupe consistant en un trouble neurodégénératif causé par le stress oxydatif, l'épilepsie et la néphropathie diabétique.
